Europäisches Patentamt

European Patent Office

Office européen des brevets

(11) Publication number: **0 068 417**
**B1**

(12) # EUROPEAN PATENT SPECIFICATION

(45) Date of publication of patent specification: **05.06.85**

(51) Int. Cl.⁴: **F 21 V 29/00, A 61 B 1/06**

(21) Application number: **82105472.3**

(22) Date of filing: **22.06.82**

(54) **Endoscope light source apparatus.**

(30) Priority: **23.06.81 JP 96874/81**
**23.06.81 JP 96879/81**

(43) Date of publication of application:
**05.01.83 Bulletin 83/01**

(45) Publication of the grant of the patent:
**05.06.85 Bulletin 85/23**

(84) Designated Contracting States:
**AT BE CH DE FR GB IT LI NL SE**

(56) References cited:
**DE-A-2 532 946**
**GB-A-1 393 699**
**US-A-3 638 013**

(73) Proprietor: **OLYMPUS OPTICAL CO., LTD.**
**43-2, 2-chome, Hatagaya Shibuya-ku**
**Tokyo 151 (JP)**

(72) Inventor: **Hosono, Saburo**
**231-1, Takahata**
**Hino-shi Tokyo (JP)**

(74) Representative: **KUHNEN & WACKER**
**Patentanwaltsbüro**
**Schneggstrasse 3-5 Postfach 1729**
**D-8050 Freising (DE)**

Courier Press, Leamington Spa, England.

## Description

This invention relates to a light source apparatus for supplying an illumination light to an endoscope light guide.

From US—A—3 638 013 an endoscope light source apparatus is known comprising a light source, a case which contains the light source, said light source being provided with an inlet connected to a gas source that is, with this known apparatus, formed by the ambient air. The influx into the case of the ambient air as cooling air is achieved by means of fans. Furthermore, the case comprises an outlet for releasing air from the case due to pressure differences between the interior of the case and the outer environments provided by the fan, said light source being provided between said gas inlet and said gas outlet which is, with this known apparatus, formed by louvered openings that stay open irrespective of the operation conditions.

A main disadvantage of this arrangement is that for cooling the light source, air is blown through the case. Air, however, is a mixture of gases including oxygen which in connection with inflammable gas, which may often occur in the atmosphere of endoscopes used for industrial purposes, can cause problems together with a very high temperature of the light source possible leading to an explosion. With the apparatus known from US—A—3 638 013, however, air not only penetrates through the inlet but also through the outlet of the case since these outlets are always open, i.e. even if the apparatus is switched off. So, even if no inflammable gas has penetrated into the case by the fan during operation, inflammable gas components may penetrate through the outlet thereby generating an explosive gas mixture being ignited when, after restarting of the light source apparatus, air containing oxygen is fed through the inlet of the case.

The problem underlying the invention is therefore to provide an endoscope light source apparatus according to the first portion of claim 1 that is able to prevent a creation of an explosive gas mixture both during operating mode and non-operating mode.

The solution of this problem is achieved by the features of the characterizing portion of claim 1.

By providing means closing the gas passage on the side of the outlet of the case between the interior and exterior of the case at normal pressure inside the case, i.e. when there is no pressure difference between the interior and the exterior of the case, any penetration of inflammable gases both through the inlet and the outlet is securely prevented.

So, even if the endoscope light source apparatus according to the invention is used in an atmosphere containing inflammable gas, there is no danger of penetrating of this gas into the case and consequently the occurrence of an explosion is successfully suppressed.

The dependent claims 2 to 4 contain preferred embodiments of the invention.

This invention can be more fully understood from the following detailed description when taken in conjunction with the accompanying drawings, in which:

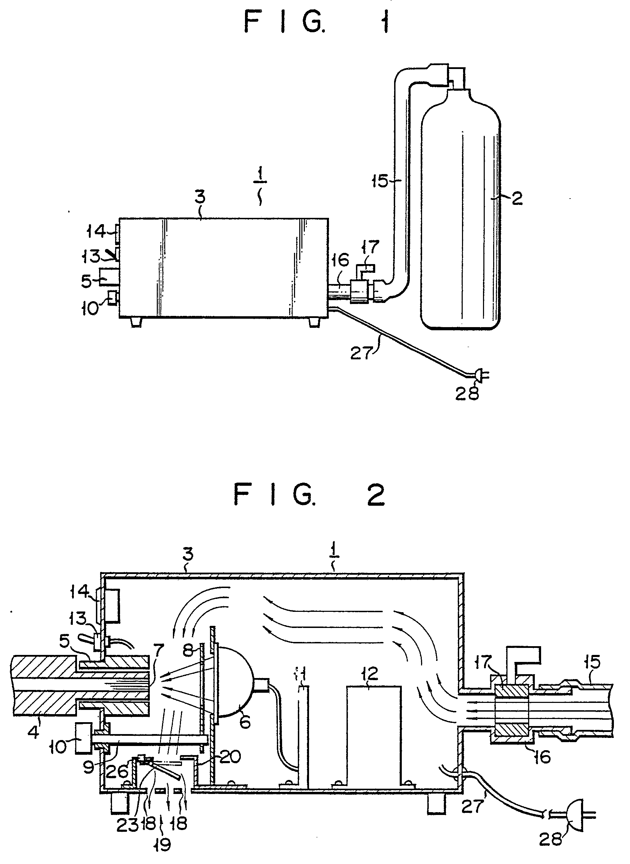

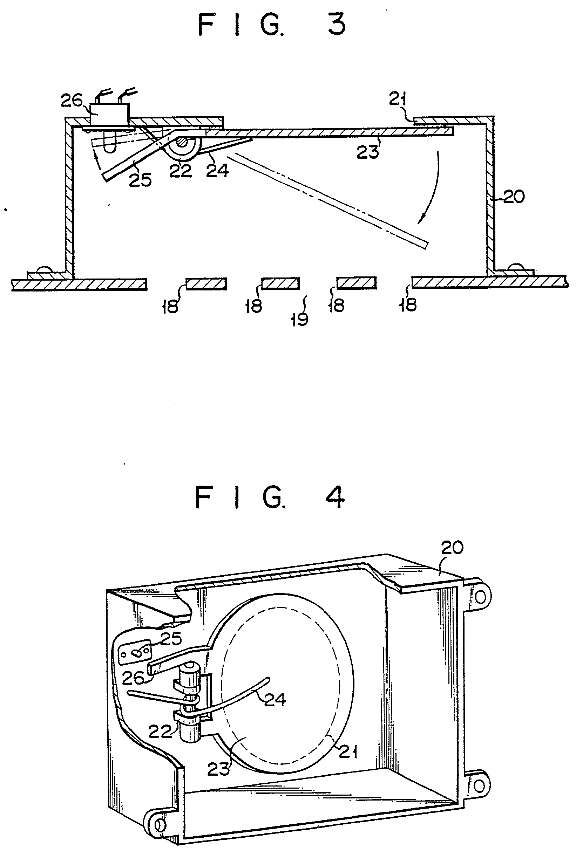

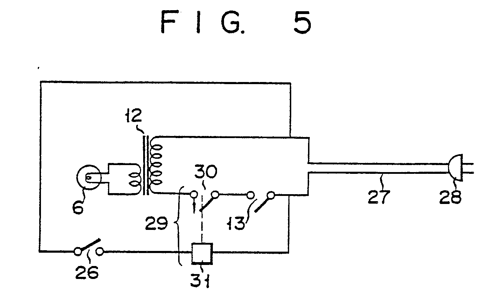

Figs. 1 to 5 show an endoscope light source apparatus in which Fig. 1 is a side view of a light source apparatus connected to a gas cylinder, Fig. 2 is a sectional view of the whole light source apparatus, Fig. 3 is an enlarged sectional view of air outlets, Fig. 4 is an oblique view of a pressure-sensing mechanism, and Fig. 5 is a drive circuit of a lamp; and

Description is now given with reference to Figs. 1 to 5 of an endoscope light source apparatus according to a first embodiment of this invention.

Referring to Fig. 1, reference numeral 1 denotes an endoscope light source apparatus. Connected to the light source apparatus 1 is a cylinder 2 acting as a gas source which holds, for example, an noninflammable gas in a compressed state. A case 3 of the endoscope light source apparatus 1 is rendered airtight. The front wall of the case 3 is penetrated by a connector inlet 5, to which an endoscope light guide connector 4 is fitted. A light source or lamp 6 is set inside of said connector inlet 5. Light beams emitted from the lamp 6 are converted to a light-receiving end face 7 of the light guide connector 4 fitted into the connector inlet 5. A light quantity-controlling shutter 8 is interposed between the lamp 6 and connector inlet 5. This light quantity-controlling shutter 8 is connected to one end of an operation rod 9 penetrating the case 3 in an airtight state. The light quantity-controlling shutter 8 is actuated by a light quantity-controlling knob 10 fitted to the opposite end of the operation rod 9. Parts such as a lamp circuit substrate 11 and transformer 12 are received in the case 3. The front outer wall of the case 3 is fitted with a power supply switch 13 and pressure gauge 14. This pressure gauge 14 detects gas pressure prevailing in the case 3 and displays the detected gas pressure.

The rear wall of the case 3 is provided with a gas inlet 16, to which a gas hose 15 of the cylinder 2 is attached. The gas inlet 16 is provided with a changeover cock 17 to open or close the gas inlet 16 as need requires. When opened, the cock 17 enables a compressed noninflammable gas to be taken into the case from the cylinder 2.

A large number of small holes 18 penetrate that portion of the bottom wall of the case 3 which lies near the front wall thereof. The inner wall of that portion of the bottom wall which is drilled with the numerous small holes 18 is provided with a support case 20 to which an intracase pressure-sensing board is fitted, and whose opening communicates with said small orifices 18. A gas passage 21 for causing the interior of said case 20 to communicate with that of the case 3 of the endoscope light source apparatus 1 is formed in the upper wall of said case 20 (Fig. 3). A pressure-sensing board 23 is provided in said case 20 in such a manner that one end of said sensing board 23 is swingably set inside of the upper wall of said case 20 by means of a hinge 22. Fitted to this hinge 22 is a torsion spring 24, one end of which is engaged with the pressure-sensing board 23, and the other end of which is engaged with the upper wall of the case 20. The torsion spring 24 urges the intracase pressure-sensing board 23 in such a direction as causes the gas passage 21 to be closed from the inside of the upper wall of the case 20, thereby providing gas outlets 19.

The end of the hinged side of the pressure-sensing board 23 is fitted with a downward projecting operation arm 25. A safety switch 26 is set inside of the upper wall of the case 20 in a state facing the free end of the operation arm 25. When the pressure-sensing board 23 swings through a prescribed angle (to the imaginary lines given in Fig. 3), then a switch 26 is closed by the free end of the operation arm 25. Conversely when released from the free end of the operation arm 25, the switch 26 is opened.

The rear wall of the case 3 of the endoscope light source apparatus 1 is penetrated by a power supply cord 27 in an airtight state. The outer end of the power supply cord 27 is fitted with a power supply plug 28.

Fig. 5 shows a power supply circuit consisting of the circuit substrate 11 and transformer 12. Connected in series to the power supply plug 28 are a power supply switch 13, the normally open contact 30 of a relay switch 29 and the primary winding of the transformer 12. Connected to the power supply plug 27 is another series circuit consisting of the safety switch 26 and a solenoid 31 of the relay switch 29. This series circuit is set in parallel to the aforementioned series circuits. The secondary winding of the transformer 12 is connected to the lamp 6.

Description is now given of the operation of an endoscope light source apparatus according to a first embodiment of this invention.

First, an endoscope light guide connector 4 is inserted into the connector inlet 5. A compressed noninflammable gas is taken into the case 3 from the gas cylinder 2 by opening the cock 17 of the gas inlet 16. When the pressure of the gas filled in the case 3 increases to a certain level, the intracase pressure-sensing board 23 swings against the urging force of the spring 24. As a result, a gas remaining in the case 3 up to this point is drawn off to the outside through the gas passage 21 and small holes 18. When a pressure in the case 3 falls below a prescribed level due to the influx of the noninflammable gas, the intracase pressure-sensing board 23 swings to a maximum extent, causing the safety switch 26 to be closed by the operation arm 25. Accordingly, the solenoid 31 of the relay switch 29 is energized to close the contact 30 of said relay switch 29, thereby rendering the subject lamp 6 operable. The level of an intracase pressure is indicated on a pressure gauge 14 ready for confirmation. When the compressed noninflammable gas is taken in, the pressure increases above the ambient air pressure, thereby preventing the influx of the ambient air. Even when, therefore, an inflammable gas happens to prevail around the case 3, said gas is not likely to enter the case 3 and give rise to an explosion.

When the power supply switch 13 is rendered conducting after the gas pressure rises above the external atmospheric pressure, then power is supplied to the lamp 6 through the already closed contact 30 and transformer 12 (Fig. 5), thereby causing the lamp 6 to be lighted. Thus, fresh streams of a noninflammable gas are always drawn into the case 3 to cool the parts within case 3, particularly the light guide connector 4, connector inlet 5 and lamp 6, thereby preventing their overheating.

When the amount of the noninflammable gas entering the case 3 decreases with a decline in pressure, then the pressure-sensing board 23 is closed by the urging force of the torsion spring 24 to shut off the gas passage 21. At this time, the safety switch 26 is actuated to render the subject light source apparatus nonconducting, and the contact 30 is opened due to the cutoff of power supply to the solenoid 31 of the relay switch 29, causing the lamp 6 to be extinguished. Therefore, the danger is forestalled that the lamp 6 continues to be lighted even when a noninflammable gas does not flow in, possibly resulting in the overheating damage of, for example, a light guide.

Further, even if carried into an overheated region or electric circuit, an inflammable gas is prevented from starting an explosion.

An endoscope light source apparatus according to the embodiment of the invention can be rendered compact, because an electrically driven fan is not provided in the case 3. Of course, it is possible to install the electrically driven fan in order to assure the thorough cooling of the whole light source apparatus by stirring gas steams in the case 3.

The source of the noninflammable gas does not need to be limited to the previously described gas cylinder. But it is possible to use an air compressor commonly installed in a plant as means for supplying the noninflammable gas.

The endoscope light source apparatus of the invention has the advantages that since the case is filled with a noninflammable gas with a pressure higher than the atmospheric pressure, an inflammable gas surrounding the case is prevented from being carried thereinto, and consequently suppressing the occurrence of an explosion; and the case is effectively cooled by gas streams flowing therethrough.

**Claims**

1. An endoscope light source apparatus (1) comprising a light source (6) for supplying light to an endoscope, a case (3) which contains the light source (6) which includes an inlet (16) connected to a gas source, allowing for the influx of a cooling gas into the case (3), an outlet (19) enabling the gas remaining in the case (3) to be drawn out by a difference between the pressure inside and outside the case (3), said light source (6) is provided between said gas inlet (16) and said gas outlet (19), and said gas outlet (19) is formed of a large number of holes (18) penetrating the case (3) characterized by a support case (20) housed in the case (3) to enclose said numerous penetrating holes (18) and provided with a gas passage (21) for effecting communication between the interior and the exterior of the case (3), a board (23) which is fitted to the inner wall of the support case (20) and made movable between a position closing the gas passage (21) and a position opening said gas passage (21), and urging means (24) for normally urging said board (23) to a gas passage-closing position; and, when the pressure of the gas inside the case (3) exceeds a prescribed level, then said board (23) is shifted to a position for opening said gas passage (21) against the force of the urging means (24), thereby causing the gas in the case (3) to flow out through said gas passage (21) and said penetrating holes (18), and wherein said cooling gas is noninflammable.

2. The light source apparatus according to claim 1, characterized in that said gas source comprises a cylinder (2) holding said cooling gas in a compressed state.

3. The light source apparatus according to claim 1 or 2, characterized by compressing a circuit (11, 12) for actuating the light source (6), and a switch (26) which is operated by said board (23) when moved to a position for opening the gas passage (21) and allows for the lighting of the lamp by said lamp-actuating circuit.

4. A light source apparatus according to claim 3, characterized in that said light source-actuating circuit (11, 12) is provided in the case (3) between said gas inlet (16) and said gas outlet (19).

## Patentansprüche

1. Lichtquellenanordnung (1) an einem Endoskop, mit einer Lichtquelle (6) für das Einstrahlen von Licht in ein Endoskop, einem Gehäuse (3), in dem sich die Lichtquelle (6) befindet und das einen an eine Gasquelle angeschlossenen Einlaß (16) aufweist, durch den Kühlgas in das Gehäuse (3) eintreten kann, einem Auslaß (19), durch den das in dem Gehäuse (3) verbleibende Gas infolge eines Unterschieds zwischen dem innerhalb und außerhalb des Gehäuses (3) herrschenden Druck abgezogen werden kann, wobei die Lichtquelle (6) sich zwischen dem Gaseinlaß (16) und dem Gasauslaß (19) befindet und der Gasauslaß (19) aus einer großen Zahl von Öffnungen (18) besteht, die in das Gehäuse (3) geschnitten sind, dadurch gekennzeichnet, daß in dem Gehäuse (3) ein Tragkasten (20) vorgesehen ist, der die zahlreichen Durchtrittsöffnungen (18) verdeckt und mit einem Gasdurchlaß (21) zum Herstellen einer Verbindung zwischen dem Innenraum des Gehäuses (3) und dem Außenraum ausgestattet ist, ferner mit einer an der Innenwand des Tragkastens (20) angebrachten Platte (23), die zwischen einer Stellung zum Verschließen des Gasdurchlasses (21) und einer Stellung zum Offenhalten des Gasdurchlasses (21) hin und her bewegbar ist, und schließlich mit einer kraftausübenden Einrichtung (24), von der die Platte (23) normalerweise in eine den Gasdurchlaß verschließende Stellung gedrückt wird, während die Platte (23), wenn der Gasdruck im Inneren des Gehäuses (3) eine vorgegebene Höhe übersteigt, gegen die Wirkung der kraftausübenden Einrichtung (24) in eine den Gasdurchlaß (21) öffnende Stellung gebracht wird, was dazu führt, daß das Gas in dem Gehäuse (3) durch den Gasdurchlaß (21) und die Durchtrittsöffnungen (18) abfließt, und daß das Kühlgas unentflammbar ist.

2. Lichtquellenanordnung nach Anspruch 1, dadurch gekennzeichnet, daß die Gasquelle als das Kühlgas in verdichtetem Zustand enthaltende Flasche (2) ausgebildet ist.

3. Lichtquellenanordnung nach Anspruch 1 oder 2, dadurch gekennzeichnet, daß eine Schaltung (11, 12) zum Speisen der Lichtquelle (6) vorgesehen ist sowie ein Schalter (26), der von der Platte (23), wenn diese sich in eine Stellung zum Öffnen des Gasdurchlasses bewegt, betätigt wird und die Lampe über die Lampenspeiseschaltung einschaltet.

4. Lichtquellenanordnung nach Anspruch 3, dadurch gekennzeichnet, daß die Lichtquellenspeiseschaltung (11, 12) sich in dem Gehäuse (3) zwischen dem Gaseinlaß (16) und dem Gasauslaß (19) befindet.

## Revendications

1. Dispositif formant une source lumineuse (1) comprenant une source lumineuse (6) pour fournir la lumière à un endoscope, un boîtier (3) contenant la source lumineuse (7), ce dernier présentant une entrée (16) reliée à une source de gaz permettant ainsi l'entrée d'un gaz de refroidissement dans le boîtier (3), une sortie (19) permettant au gaz restant dans le boîtier (3) d'être aspiré par la différence entre la pression à l'intérieur et à l'extérieur du boîtier (3), ladite source lumineuse (6) étant disposée entre ladite entrée de gaz (16) et la sortie de gaz (19) ladite sortie de gaz (19) étant formée par un grand nombre de perforations (18) pénétrant à travers le boîtier (3), caractérisé par un boîtier de support (20) placé dans le boîtier (3) pour renfermer lesdites perforations nombreuses (18) et étant pourvu d'un passage de gaz (21) pour effectuer la communication entre l'intérieur et l'extérieur du boîtier (3), une plaque (23) montée sur la paroi intérieure du boîtier de support (20) et rendue mobile entre une position fermant le passage de gaz (21) et une position d'ouverture dudit passage de gaz (21) et des moyens de pression (24) destinés à presser normalement ladite plaque (23) vers une position de fermeture du passage; et par ce que lorsque la pression du gaz à l'intérieur du boîtier (3) dépasse un niveau prédéterminé, ladite plaque (23) est déplacée vers une position d'ouverture dudit passage de gaz (21) contre la force dudit moyen de pression (24), ce qui fait que le gaz dans le boîtier (3) sort par ledit passage de gaz (21) et lesdites perforations et par ce que ledit gaz de refroidissement n'est pas inflammable.

2. Dispositif formant une source lumineuse selon la revendication 1, caractérisé par le fait que ladite source de gaz comprend un cylindre (2) qui maintient ledit gaz de refroidissement dans un état comprimé.

3. Dispositif formant une source lumineuse selon les revendications 1 ou 2, caractérisé par le fait qu'il comprend un circuit (11, 12) pour commander la source de lumière (6) et un interrupteur (26) qui est déplacée vers une position destinée à ouvrir le passage de gaz (21) et qui permet d'allumer la lampe par ledit circuit d'actuation de la lampe.

4. Dispositif formant une source de lumineuse selon la revendication 3, caractérisé par le fait que ledit circuit d'actuation de la source de lumière (11, 12) est placé dans le boîtier (3) entre ladite entrée de gaz (16) et ladite sortie de gaz (19).

0 068 417

# F I G. 1

# F I G. 2

F I G. 3

F I G. 4

# F I G. 5